(19) Europäisches Patentamt
European Patent Office
Office européen des brevets

(11) EP 3 183 952 A1

(12) # EUROPEAN PATENT APPLICATION
published in accordance with Art. 153(4) EPC

(43) Date of publication:
**28.06.2017 Bulletin 2017/26**

(21) Application number: **15833855.8**

(22) Date of filing: **30.06.2015**

(51) Int Cl.:
***A01G 7/00*** *(2006.01)* ***G06Q 50/02*** *(2012.01)*

(86) International application number:
**PCT/JP2015/068838**

(87) International publication number:
**WO 2016/027566 (25.02.2016 Gazette 2016/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **20.08.2014 JP 2014167840**

(71) Applicant: **Sony Corporation**
**Tokyo 108-0075 (JP)**

(72) Inventor: **YOSHIDA, Kaoru**
**Tokyo 141-0022 (JP)**

(74) Representative: **Witte, Weller & Partner Patentanwälte mbB**
**Postfach 10 54 62**
**70047 Stuttgart (DE)**

(54) **INFORMATION PROCESSING DEVICE, INFORMATION PROCESSING METHOD, AND PROGRAM**

(57) [Object] To obtain an appropriate index relating to a method for obtaining a desired crop by appropriately modeling characteristics of a crop.

[Solution] Provided is an information processing apparatus including a condition input unit configured to accept input of conditions relating to an effect of a crop on an ingester, and a model referring unit configured to specify, by referring to a model in which the effect is associated with a factor relating to the crop or the ingester, the factor for satisfying the conditions, an information processing method, or a program.

**FIG. 7**

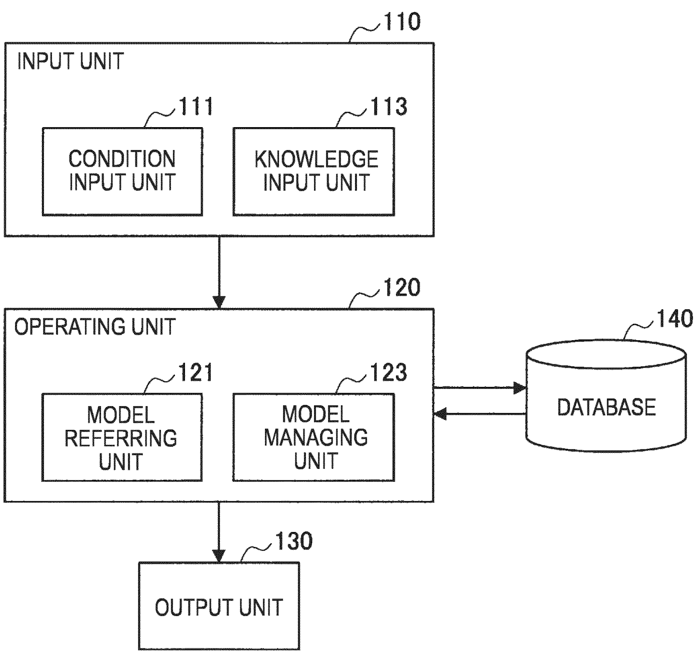


EP 3 183 952 A1

## Description

### Technical Field

The present disclosure relates to an information processing apparatus, an information processing method, and a program.

### Background Art

Various technologies have been developed concerning the analysis of components of a crop. For example, genomic analysis and proteomic analysis are known as methods for exhaustively analyzing genes and proteins within a living body. Further, in recent years, metabolome analysis which exhaustively analyzes metabolic substances within a living body is put into practical use. As a technology relating to such a method, for example, Patent Literature 1 discloses a method for efficiently identifying a metabolic substance in the metabolome analysis.

### Citation List

### Patent Literature

Patent Literature 1: JP 2009-20037A

### Disclosure of Invention

### Technical Problem

However, in an exhaustive analysis method such as metabolome analysis, enormous kinds of components are to be measured. Therefore, because characteristics of a crop which becomes a sample is often explained using, for example, a peak value relating to a specific component, it is not easy to recognize overall characteristics. Accordingly, the analysis results are merely applied to an individual specific case, and the analysis method cannot be said as sufficient means for extracting characteristics of a crop, which is more generalized and practical.

Therefore, the present disclosure proposes new and improved information processing apparatus, information processing method and program which enable an appropriate index relating to a method for obtaining a desired crop to be obtained by appropriately modeling characteristics of a crop.

### Solution to Problem

According to the present disclosure, there is provided an information processing apparatus including: a condition input unit configured to accept input of conditions relating to an effect of a crop on an ingester; and a model referring unit configured to specify, by referring to a model in which the effect is associated with a factor relating to the crop or the ingester, the factor for satisfying the conditions.

According to the present disclosure, there is provided an information processing method including: accepting input of conditions relating to an effect of a crop on an ingester; and specifying by a processor, by referring to a model in which the effect is associated with a factor relating to the crop or the ingester, the factor for satisfying the conditions.

According to the present disclosure, there is provided a program causing a computer to execute: a function of accepting input of conditions relating to an effect of a crop on an ingester; and a function of specifying, by referring to a model in which the effect is associated with a factor relating to the crop or the ingester, the factor for satisfying the conditions.

For example, concerning crops used as food or medicine, there are a lot of cases where the relationship between effects and factors relating to a crop or an ingester is partially clear as a result of, for example, the analysis of components, as a result of experiment or examination, or through empirical knowledge. By constructing a model by integrating such knowledge and further updating the model as needed, it can be easy to appropriately model characteristics of a crop and obtain an appropriate index relating to a method for obtaining a desired crop.

### Advantageous Effects of Invention

As described above, according to the present disclosure, it is possible to obtain an appropriate index relating to a method for obtaining a desired crop by appropriately modeling characteristics of a crop.

Note that the effects described above are not necessarily limitative. With or in place of the above effects, there may be achieved any one of the effects described in this specification or other effects that may be grasped from this specification.

Brief Description of Drawings

[FIG. 1] FIG. 1 is a diagram illustrating an example of a system according to one embodiment of the present disclosure.
[FIG. 2] FIG. 2 is a diagram illustrating an example of a model constructed in one embodiment of the present disclosure.
[FIG. 3] FIG. 3 is a diagram illustrating an example of classification relating to components of a crop defined in one embodiment of the present disclosure.
[FIG. 4] FIG. 4 is a diagram illustrating an example of association between component classification of a crop and agricultural factors in one embodiment of the present disclosure.
[FIG. 5] FIG. 5 is a diagram illustrating an example of association between component classification of a crop and agricultural factors in one embodiment of the present disclosure.
[FIG. 6] FIG. 6 is a diagram illustrating an example of association between component classification of a crop and agricultural factors in one embodiment of the present disclosure.
[FIG. 7] FIG. 7 is a block diagram illustrating an example of a software functional configuration in one embodiment of the present disclosure.
[FIG. 8] FIG. 8 is a diagram illustrating an example of raw data of absorbance measurements.
[FIG. 9] FIG. 9 is a diagram illustrating an example where the raw data illustrated in FIG. 8 is normalized and the dimension is reduced by expanding the unit width to 100 nm.
[FIG. 10] FIG. 10 is a diagram illustrating an example where the raw data illustrated in FIG. 8 is normalized and the dimension is reduced by expanding the unit width to 50 nm.
[FIG. 11] FIG. 11 is a diagram illustrating an example where another raw data of absorbance measurements is normalized and the dimension is reduced by expanding the unit width to 100 nm.
[FIG. 12] FIG. 12 is a block diagram illustrating a hardware configuration example of an information processing apparatus according to the embodiment of the present disclosure.

Mode(s) for Carrying Out the Invention

Hereinafter, a preferred embodiment of the present disclosure will be described in detail with reference to the appended drawings. In this specification and the appended drawings, structural elements that have substantially the same function and structure are denoted with the same reference numerals, and repeated explanation of these structural elements is omitted.
Note that description will be provided in the following order.

1. System configuration
2. Model construction
2-1. Outline of model
2-2. Example of classification relating to components of crop
2-3. Example of agricultural factors
2-4. Example of association between component classification of crop and agricultural factors
3. Functional configuration example
4. Example of utilization of component analysis results
4-1. Generation of database
4-2. Utilization of database
5. Modified example
6. Hardware configuration
7. Supplement

(1. System Configuration)

First, an example of a system configuration according to one embodiment of the present disclosure will be described.
FIG. 1 is a diagram illustrating an example of a system according to one embodiment of the present disclosure. The system according to one embodiment of the present disclosure includes one or more information processing apparatuses. In the example illustrated in FIG. 1, the system 10 includes an input/output apparatus 12, an arithmetic apparatus 14 and a storage apparatus 16. These apparatuses are interconnected through a network such as, for example, the Internet. It should be noted that, in other examples, any one or all of these apparatuses may be implemented by being integrated into a single information processing apparatus. Alternatively, any one of these apparatuses may be implemented by being distributed into a plurality of information processing apparatuses.

The input/output apparatus 12 accepts input/output of information for the system 10. The input/output apparatus 12 is a terminal apparatus such as, for example, a personal computer, a tablet and a smartphone, and does not have to be a dedicated terminal for the system 10. For example, the input/output apparatus 12 may be provided at enterprises, research institutes, or the like, and used for business purposes or used for personal purposes. The input/output apparatus 12 can include a plurality of terminal apparatuses to be used by different users. Further, the input/output apparatus 12 may be implemented by being distributed into terminal apparatuses which are different for each type of information to be input/output. For example, inputting definitions of effects of a crop and factors relating to a crop or an ingester which will be described later, and/or knowledge for generating or updating a model in which effects are associated with factors can be executed by a terminal apparatus used by a manager of the system 10 for business purposes. On the other hand, inputting conditions in the case of specifying a factor for satisfying conditions relating to an effect by utilizing the above-described model or outputting the specified factor may be executed by the same terminal apparatus used for business purposes as described above or may be executed by a terminal apparatus used for personal purposes.

The arithmetic apparatus 14 executes various kinds of operations in the system 10. For example, based on conditions relating to an effect input at the input/output apparatus 12, the arithmetic apparatus 14 makes the input/output apparatus 12 output a factor relating to a crop or an ingester for satisfying the conditions with reference to a model constructed at the storage apparatus 16. Further, the arithmetic apparatus 14 may execute an operation for generating or updating a model. For example, the arithmetic apparatus 14 may newly generate a model based on knowledge input at the input/output apparatus 12, for example, analysis results of components of a crop, or the like, and store the model in the storage apparatus 16 or update the already generated model. It should be noted that, in the case where a model constructed outside is loaded into the storage apparatus 16 as initial data, the arithmetic apparatus 14 does not have to execute the operation for generating a model. Further, in the case where a model is updated separately (for example, in the case where data of the updated model is loaded into the storage apparatus 16 each time), the arithmetic apparatus 14 does not have to execute the operation for updating a model.

In the storage apparatus 16, various kinds of data used in the system 10 is stored. For example, in the storage apparatus 16, data of a model in which effects of a crop on an ingester are associated with factors of the crop or the ingester is stored. As described above, the model may be generated or updated through an operation at the arithmetic apparatus 14 based on the knowledge input from the input/output apparatus 12. It should be noted that details of the model will be described later. Further, in the storage apparatus 16, other data to be utilized in the system 10 may be stored. For example, data which defines effects or factors defined in the above-described model may be stored in the storage apparatus 16.

(2. Model Construction)

A model constructed in the present embodiment will be further described next. As described above, in the present embodiment, a model in which effects of a crop on an ingester are associated with factors relating to the crop or the ingester is constructed.

(2-1. Outline of model)

FIG. 2 is a diagram illustrating an example of a model constructed in one embodiment of the present disclosure. In the example illustrated in FIG. 2, the model is defined by translators T1 to T3 and mapping engines M1 and M2. It should be noted that, in the illustrated example, the model includes components of a crop (element U) as intermediate factors respectively associated with effects (element V) and agricultural factors (element W) for providing the crop. Each element will be further described.

The element V (V1 to Vm) of the effect is used to, for example, input conditions relating to an effect at the input/output apparatus 12. For example, when the effect is input by being selected from a list, the element V can include types of effects displayed at the list. Further, for example, when the effect is input as a text, the element V can include various keywords which can be extracted from the text. The translator T1 converts the element V of the effect into a class G (G1 to Gq). The class G corresponds to characteristics of the effect extracted from the element V.

More specifically, the element V of the effect may indicate a state which is realized at an ingester of a crop, such as, for example, "nutritional fortification" and "improvement in sensitivity to cold temperatures" or may indicate an attribute of the ingester, such as, for example, an infant and an elderly person. Further, the element V of the effect may indicate whether a crop is ingested as food or ingested as supplement. That is, in the present embodiment, the element V of the effect can correspond to wide concept including conditions at the ingester side, for example, "who", "under what kind of conditions a crop is ingested" and "what kind of state the ingester is to be put into".

Further, as well as positive effects, negative effects, for example, unhealthy, difficulty in ingestion, or the like, may be defined. When a negative effect is input as conditions, for example, it is possible to specify a crop which should be avoided or a factor of the ingester.

Here, while the element V is defined based on, for example, name, or the like, of the effect typically recognized by a user so as to allow easy inputting, the class G is defined to associate effects which will be described later with components of a crop. Therefore, definition of the class G and a configuration of the translator T1 which converts the element V into the class G can be changed as needed according to the class F of the component and a configuration of the mapping engine M1. Further, the class G does not necessarily have to have a label which can be recognized by a user. That is, the class G may be an abstract class which does not necessarily correspond to a known name, or the like.

It should be noted that the element V may be classified in a plurality of classes G in an overlapped manner. That is, the element V having a plurality of characteristics can be converted into a plurality of classes G corresponding to the respective characteristics by the translator T1. Therefore, for example, even if one element V of the effect is input, by converting the element V into a plurality of classes G and associating these classes G with classes F having different components by the mapping engine M1, as a result, an agricultural factor including combination of a plurality of breeds of crops and growth environments can be specified.

The element U (U1 to Ul) of components of a crop can be defined as intermediate factors respectively associated with effects and agricultural factors as described above. Because effects of a crop on an ingester appear by components of the crop provided from agricultural factors in part, it is rational to define components of a crop as intermediate factors between the effects and the agricultural factors. There are already a lot of pieces of knowledge which associate components of a crop with effects. Further, the relationship between components of a crop and agricultural factors can be extracted from, for example, analysis results of components of a crop for which agricultural factors are specified.

Here, in the illustrated example, also concerning the element U of the components, the translator T2 executes the conversion into the class F (F1 to Fp). Similarly as shown with the above-described class G of the effect, the class F is also defined to associate effects with agricultural factors. Because there are enormous types of components of a crop, it is not easy to associate individual components with effects and agricultural factors. Therefore, in the present embodiment, by defining the class F corresponding to characteristics of the components, association between the effects and the agricultural factors is facilitated. It should be noted that, similarly as shown with the above-described examples of the effects, the class F and a configuration of the translator T2 can be also changed as needed according to the class G of the components, the class H of the agricultural factors and the configurations of the mapping engines M1 and M2.

More specifically, for example, the class F can be defined by functions of components, a metabolic pathway and/or whether the component is a natural compound or a synthetic compound. Further, the class F may be defined based on taste or aroma provided to the crop by components. Similarly as shown with the above-described example of the class G, the element U of the components may be also classified into a plurality of classes F in an overlapped manner. It should be noted that a more specific example of classes F1 to Fp of the components will be described later.

The element W of the agricultural factors (W1 to Wn) is used, for example, when factors for satisfying conditions input at the input/output apparatus 12 are output. In the present embodiment, the agricultural factors can include a breed and a growth environment of the crop. More specifically, the growth environment of the crop can include states of fertilization, vermin/infection control, weed control, tillage, vegetation and biodiversity, a light wavelength, a light amount, a light irradiation period, climate, soil components, a soil state, a dosage amount of fertilizer or pesticide or a growing period. Further, the agricultural factors output as factors for satisfying conditions may include combination of a plurality of breeds or combination of a plurality of growth environments.

Here, in the illustrated example, also concerning the element W of the agricultural factors, the translator T3 executes the conversion into class H (H1 to Hr). The class H also corresponds to characteristics of agricultural factors extracted from the element W and is defined to associate agricultural factors with the components of the crop. The element W may be classified into a plurality of classes H in an overlapped manner. Further, similarly as shown with, for example, the above-described class G, or the like, the class H does not necessarily have to have a label which can be recognized by the user. Alternatively, the class H may have a label which can be recognized by the user and may be utilized to output agricultural factors as necessary. It should be noted that a more specific example of the class H of the agricultural factors will be described later.

The mapping engine M1 associates the class G (G1 to Gq) of the effects with the class F (F1 to Fp) of the components of the crop. As described above, this association can be defined based on, for example, already existing knowledge concerning the effects of the components of the crop. For example, the mapping engine M1 can be constructed by learning the relationship between classes using such knowledge as training data and can be further updated as needed. In this event, not only algorithm of the mapping engine M1 but also respective classes, that is, the class G of the effects and the class F of the components can be optimized so that the relationship between the effects and the components is appropriately mapped.

The mapping engine M2 associates the class F (F1 to Fp) of the components of the crop with the class H (H1 to Hr) of the agricultural factors. As described above, this association can be defined based on, for example, analysis results of the components of the crop for which the agricultural factors are specified. For example, the mapping engine M2 can be constructed by learning the relationship between classes using knowledge such as the analysis results of the components as training data and can be further updated as needed. In this event, not only algorithm of the mapping engine M2 but also respective classes, that is, the class F of the components and the class H of the agricultural factors can be optimized so that the relationship between the components and the agricultural factors is appropriately mapped.

One example of the model constructed in one embodiment of the present disclosure has been described above. It should be noted that, in the present embodiment, a model in which the effects of the crop on the ingester are associated with factors relating to the crop or the ingester is constructed. Therefore, while, in the above-described example, a model in which the effects are associated with factors relating to the crop (agricultural factors) is constructed, in other examples, a model in which effects are associated with factors relating to the ingester or a model in which effects are associated with both factors relating to a crop and factors relating to the ingester may be constructed. In such a case, also concerning the factors relating to the ingester, similarly as shown with the above-described effects, components and agricultural factors, conversion into the class by the translator can be executed. The factors relating to the ingester can include, for example, behavior factors of the ingester. Further, as the factors relating to the crop, not limited to the above-described agricultural factors, but various other factors relating to the crop can be defined. Therefore, in the present embodiment, the model does not necessarily have to include components of the crop as intermediate factors.

(2-2. Example of classification relating to components of crop)

An example of classification relating to components of a crop which can be defined in the model as described above will be further described next. While the components of the crop do not have to be necessarily included in the model, there is a case where the components of the crop are useful as intermediate factors respectively associated with the effects of the crop on the ingester and the agricultural factors for providing the crop.

FIG. 3 is a diagram illustrating an example of classification relating to the components of the crop defined in one embodiment of the present disclosure. In the illustrated example, the components of the crop are classified based on a metabolic pathway. In this case, as is clear from some examples which will be described below, it can be said that the components of the crop are classified based on functions. Further, in the illustrated example, the components of the crop are classified according to whether the component is a natural compound (Natural, map 1100) or a synthetic compound (Synthetic, map 1200). Each of regions of illustrated classification, for example, corresponds to a class of the component of the crop illustrated in FIG. 2. An example of the illustrated classification will be described below with an example of effects with which some classification is associated.

First, in "Organic acids" located at the center of the map 1100 of the natural compound (Natural), organic compounds involved with a citric acid cycle (TCA cycle) and a glyoxylate cycle which is the center of a biochemical pathway are classified. Further, components classified into "Carbohydrates" located at the left side in the drawing seen from the center, "Nucleic acids & Nucleotides" and "Amino acids & Peptides" located at the upper side, and "Fatty acids" located at the lower side are located so that smaller compounds (monomers) are located closer to the center and larger compounds (polymers) are located farther from the center when the components are further classified. Here, decomposition reaction (catabolism) is required to transform a larger compound into a smaller compound, and synthesis reaction (anabolism) is required to transform a smaller compound into a larger compound. Generally, it is known that a smaller compound tends to be absorbed into a body with less biological reaction.

Here, as the relationship between small compounds such as components included in the above-described classification, organic acids, glucose which is monosaccharide, nucleic acids and amino acids, and effects, it is known that these components are compounds "which can be used immediately". According to such knowledge, this component classification is associated with an effect useful for a "person with a weak constitution", a "person having a weak stomach and with weak digestion", a "person who plays sports or overworks the body and who needs nutritional support immediately", or the like.

On the other hand, it is known that a polymer or a long-chain compound located at the outer side of the map 1100, for example, polysaccharide or long-chain lipids (such as Sphingolipids) such as starch is a "nutrient which is accumulated for growth in the future or for environmental change as well as current biological activity". Therefore, this component classification is associated with the effect useful for a "person who requires nutritional support in the long term", a "marathon runner before taking part in a marathon", or the like. However, it is known that cellulose which is an origin of dietary fiber among polysaccharide requires large energy for decomposition reaction. Therefore, for example, in the case where the ingester is an "infant with a weak constitution who has an undeveloped metabolic function" or an "elderly person whose metabolic function declines", cellulose as a component of a crop is not recommended.

At the right side in the drawing seen from the center in the map 1100, at the position closer to the center, a region of a chelating agent holding a metal ion, pigments having light absorption characteristics and a redox agent involving oxidation-reduction is located ("Carotenoids", "Tetrapyrroles" and "Phenyl propanoids"). Therefore, this component classification is associated with an effect useful for a "person who lacks mineral", a "person who is out of shape", or the like.

Further, at the upper right side in the drawing seen from the center, at the outer side far from the center in the map 1100, a region of "Alkaloids" is located. Alkaloids include a lot of compounds adding bitter taste as taste of a crop. Because most of the higher animals including human recognize bitter taste as a poison, when a crop having bitter taste is ingested, it is known that it is determined that a poison enters the body, and an egestion function works. Therefore, the component classification of "Alkaloids" is associated with an effect of egestion of waste from the body, so-called, detoxification. However, this classification is not recommended to an infant with a weak constitution or an elderly person who is out of shape.

At the lower right side in the drawing seen from the center, at the outer side far from the center in the map 1100, most of the components classified as "Hormones & Transmitters" and "Steroids" are compounds involving endocrine function, immune function and reproductive function. Therefore, it is necessary to pay attention when this component classification is ingested.

On the other hand, crops containing components included in the map 1200 of the synthetic compounds (Synthetic) and components classified as "Synthetic Antibiotics", "Pesticides & Herbicides", "Additives & Preservatives" and "Carcinogens" at a great rate are not recommended except in the case where these components are associated with other useful effects (for example, in the case where these components are used as medicine).

It should be noted that, particularly, concerning secondary metabolic substances, there is a case where functions are common or similar although the substances are not close to each other in the arrangement along the metabolic pathway as in the map 1100. That is, there is a case where the arrangement in the map 1100 cannot necessarily sufficiently express the functions of the secondary metabolic substances. To cope with such a case, for example, the map 1100 expressed on a plane in the illustrated example may be sterically expressed. Alternatively, also concerning secondary metabolic substances classified as natural compounds, characteristics indexes which do not depend on the metabolic pathway as expressed with the map 1200 may be provided separately.

Further, in the illustrated example, in addition to the maps 1100 and 1200, unclassified category 1300 is defined. The unclassified category 1300 includes components which do not contribute to association between effects and agricultural factors. For example, even if the components are identified in the analysis of the components, or the like, components which do not contribute to association between effects and agricultural factors can be classified as the unclassified category 1300.

It should be noted that, to classify components as in the above-described example, it is possible to utilize an already provided metabolic pathway map and data such as compound classification as appropriate. Therefore, details of the metabolic pathway and a method for specifying functions of metabolic substances will not be described in the present specification.

Here, for example, in the data of compound classification, there is a case where two or more functions are defined for one compound. In this case, for example, it is also possible to select one function using some criterion and classify the components exclusively. Alternatively, it is also possible to locate the components at a plurality of locations on the map so as to correspond to the plurality of functions and classify the components exclusively.

It should be noted that, in the above-described example, an example where component classification is not recommended has been described along with the example of effects (a "person with a weak constitution", a "person having a weak stomach and with weak digestion") with which some component classification is associated. Also in such a case where specific component classification is not recommended, the function may be expressed with the class of the effect (may be expressed as negative functions). By expressing the functions in this manner in the model, it is possible to specify not only factors corresponding to the components to be ingested but also factors corresponding to components which should be avoided from being ingested.

(2-3. Example of agricultural factors)

Table 1 is a diagram illustrating an example of agricultural factors defined in one embodiment of the present disclosure. In the example indicated in the table, three farming methods of a conventional farming method (Conventional), an organic farming method (Organic) and a natural farming method (Natural) are indicated as combination of states of Fertilization, Vermin/Infection Control, Weed Control, Tillage, Vegetation and Biodiversity.
[Table 1]

**Table 1: Farming method and Agricultural factor**

| Farming method | Conventional | Organic | Natural |
|---|---|---|---|
| Fertilization | Chemical/ Microbes | Compost/ Microbes | None |

(continued)

| Farming method | Conventional | Organic | Natural |
|---|---|---|---|
| Vermin/Infection Control | Pesticides | Plant Extracts/ Antagonists/ Field design | Field design |
| Weed Control | Pesticides | Mow & Leave | None |
| Tillage | YES | YES | NO |
| Vegetation | Mono | Mono/Companion | Companion |
| Biodiversity | Small | Medium | Large |

In the above-described example, for example, the respective states of fertilization, vermin/infection control, weed control, tillage, vegetation and biodiversity may be located as elements of the agricultural factors (element W of the agricultural factor in the example in FIG. 2), and the farming methods (conventional, organic and natural) may be located as classification of the agricultural factors (the class H in the example in FIG. 2). This copes with a case where the relationship between the components of the crop and the agricultural factors is often explained by expressing characteristics of the agricultural factors using a farming method.

It should be noted that the combination of the farming methods and the agricultural factors described in the above-described table is merely one example. In other examples, the above-described farming methods may be defined by combination of other agricultural factors. Further, in other examples, the agricultural factors may be expressed using farming methods other than those described above, for example, hydroponic culture, a symbiotic farming method, a forest farming method, or the like.

(2-4. Example of association between component classification of crop and agricultural factors)

Association between characteristics indexes and agricultural factors in the present embodiment will be further described next.

FIG. 4 to FIG. 6 are diagrams, each illustrating an example of association between the component classification of a crop and the agricultural factors in one embodiment of the present disclosure. In the illustrated example, the components of the crop are classified according to the maps 1100 and 1200 and the category 1300 described above with reference to FIG. 3. Further, in the illustrated example, the agricultural factors are expressed using classification of the conventional farming method (Conventional), the organic farming method (Organic) and the natural farming method (Natural) described above with reference to Table 1. In FIG. 4 to FIG. 6, for each of the conventional farming method, the organic farming method and the natural farming method, content (%) of metabolic substances extracted through metabolome analysis of a plant (cabbage) which is a sample is expressed using heat maps. It should be noted that, while gradation of the heat maps is set low for convenience of printing, actually, the heat maps can be expressed with further higher gradation.

By referring to these heat maps, it is possible to specify agricultural factors for providing a crop which satisfies conditions concerning conditions of component classification corresponding to conditions relating to the effects, for example, "having high content of alkaloids (the region 1101 in the map 1100)" and "not containing antibiotics (the region 1103 in the map 1100 and the region 1201 in the map 1200)".

It should be noted that data of the model actually stored in the storage apparatus 16 can take a free form regardless of the illustrated example. For example, data for drawing the map or the category as illustrated in the drawings may be held in the database. In this case, for example, as a user interface when input of the conditions is accepted at the input/output apparatus 12 or when the specified agricultural factor is output, the map or the category as illustrated in FIG. 3 to FIG. 6 may be displayed.

Alternatively, data for drawing the map or the category as illustrated in the drawings do not have to be held. More specifically, the data may be described as a table which associates the component classification of the crop indicated by the regions in the map 1100 and 1200 and the category 1300 with content for each agricultural factor included in each classification (which may be expressed using a farming method). In this case, a user interface when input of conditions is accepted at the input/output apparatus 12 or when the specified agricultural factor is output can be expressed in a text format or in a table form.

Some examples of the relationship between the characteristic indexes and the agricultural factors illustrated by data in FIG. 4 to FIG. 6 will be described below.

Among the above-described three data, in the example of the organic farming method illustrated in FIG. 5, characteristic distribution of the metabolic substances can be seen compared to the other two farming methods. For example, in the organic farming method, content of amino acids and peptides (the region 1105 in the map 1100) is higher than that in the other farming methods. On the other hand, in the organic farming method, antibiotics (the region 1103 in the map 1100 and the region 1201 in the map 1200) which are not detected in the other farming methods are detected. While such results indicate that the organic farming method is effective in the case where, for example, it is desired to cultivate a crop containing much amino acids and peptides (in this case, cabbage), the results indicate that the organic farming method is not appropriate in the case where it is desired to cultivate a crop not containing antibiotics. It should be noted that it is considered that antibiotics are detected in the example of the organic farming method because antibiotics is mixed into feedstuff of farm animals which are supply sources of compost.

Further, the example of the natural farming method illustrated in FIG. 6 indicates that while content of additives and preservatives (the region 1203 in the map 1200) is lower than that in the conventional farming method, content of other synthetic compounds (the map 1200) is substantially equivalent to that in the conventional farming method. It is considered that this indicates a possibility that, also in a farm land where the natural farming method is performed, the conventional farming method of administering synthetic compounds as fertilizers or pesticides has been performed in the past.

In the present embodiment, it is possible to specify agricultural factors which should be employed or which should be avoided to, for example, provide a crop having desired characteristics by preparing a model in which classification of the components of the crop is associated with classification of the agricultural factors based on the analysis of the components of the crop as described above.

It should be noted that the component classification of the crop in the above-described example is one example, and other various characteristic indexes can be set. As already described above, the components of the crop may be classified according to the functions. More specifically, the components of the crop can be classified according to functions such as, for example, analgesics, antifebriles, immunosuppresants and antibiotics. Further, the components of the crop may be classified according to organs where functions are expressed. For example, it is possible to classify the components of the crop according to organs such as the nervous system, the immune system, the digestive system and the circulatory system. Such component classification of the crop can be performed based on, for example, existing compound classification. Similarly as shown with the above-described examples in FIG. 4 to FIG. 6, it is also possible to express the relationship between the components of the crop and the agricultural factors by the heat maps or the data based on other classification.

(3. Functional configuration example)

An example of a software functional configuration of a system in the present embodiment will be described next.

FIG. 7 is a block diagram illustrating an example of a software functional configuration in one embodiment of the present disclosure. Referring to FIG. 7, the functional configuration can include an input unit 110, an operating unit 120 and an output unit 130. The operating unit 120 refers to a database 140. The respective units will be further described below.

The input unit 110, for example, corresponds to a communication apparatus of the arithmetic apparatus 14 which receives information input at the input/output apparatus 12 or a processor such as a CPU which controls the communication apparatus. The input unit 110 includes a condition input unit 111. The condition input unit 111 accepts input of conditions relating to an effect of a crop on an ingester. The conditions relating to the effect can be, specifically, for example, "nutritional fortification" or "improvement in sensitivity to cold temperatures". As described above, such conditions may be input, for example, by choosing an item from a list or specifying as a text.

Further, the condition input unit 111 may accept input of premise factors along with the conditions relating to the effects. The premise factors can be, for example, other factors defined in association with the effects in the model as in the above-described example, specifically, components of a crop or agricultural factors, behavior factors of an ingester, or the like. For example, as the premise factors, factors such as "having less cellulose" and "not containing antibiotics" can be input. While the premise factors are the same as the conditions relating to the effects in terms of the conditions relating to the crop, the premise factors are different from the conditions relating to the effects in that the premise factors directly designate intermediate or final factors in the model. That is, the premise factors can function as constraint conditions when output (factors relating to the crop or the ingester) is specified from input (conditions relating to the effects) according to the model.

Further, the input unit 110 may include a knowledge input unit 113. The knowledge input unit 113 accepts input of knowledge relating to the effects or the factors for generating or updating a model in which the effects are associated with the factors relating to the crop or the ingester in the present embodiment. More specifically, for example, the knowledge includes scientific knowledge or data such as analysis results of the components of the crop and results of experiments or examinations relating to the relationship between the components of the crop and the effects. Further, the knowledge is not limited to scientific knowledge or data, but may include empirical or predictive knowledge or opinion. At the knowledge input unit 113, it is also possible to allow an operation for removing knowledge which is determined as incorrect as well as an operation for adding new knowledge.

The operating unit 120, for example, corresponds to a processor such as a CPU of the arithmetic apparatus 14. The operating unit 120 includes a model referring unit 121. The model referring unit 121 refers to the model by loading the data stored in the database 140. As already described above, the model associates the effects of the crop on the ingester with the factors of the crop or the ingester. The model referring unit 121 specifies the factors relating to the crop or the ingester for satisfying conditions included in the input accepted by the condition input unit 111 by referring to the model.

Here, the specified factors may include, for example, agricultural factors for providing a crop, more specifically, a breed and a growth environment of the crop. In this case, combination of a plurality of breeds (for example, combination of cabbage and carrot) and/or combination of a plurality of growth environments (for example, combination of carrot grown in Chiba prefecture and carrot grown in Hokkaido) may be specified as the agricultural factors for satisfying the conditions. Further, the specified factors may include, for example, behavior factors of the ingester. The behavior factors of the ingester, for example, relate to a lifestyle of the ingester, and, more specifically, can include factors such as sufficient sleep and appropriate exercise. The specified factors may include factors relating to the crop alone, factors relating to the ingester alone or both the factors relating to the crop and the factors relating to the ingester.

Further, at the model referring unit 121, a preference for referring to the model may be set in advance. The preference defines user preference or priority for the factors defined in the model separately from the conditions included in the input accepted at the condition input unit 111. More specifically, for example, the model referring unit 121 may set a preference relating to a natural compound and a synthetic compound and refer to the model. In this case, for example, in the case where there are a natural compound and a synthetic compound associated with a similar effect, the natural compound can be preferentially selected, and an agricultural factor for providing a crop including the natural compound can be specified. Alternatively, in the case where there is only a synthetic compound which contains a component relating to the effect included in the conditions, this may be ignored. At the model referring unit 121, preferences relating to a processing method, taste, aroma, availability, price, or the like, may be set in addition to those described above. The preference can be also set through a user operation through the input unit 110.

Further, as with the preference, a condition of the user which is automatically detected may be set as conditions for referring to the model. More specifically, for example, by acquiring a life log of the user by utilizing an acceleration sensor and a position sensor (such as a GPS), by analyzing an image of the meal ingested by the user or by detecting a condition of sleep of the user, it is possible to recognize the condition of the user. Based on the condition recognized in this manner, effects required by the user, a crop which is recommended to be ingested for realizing the effects or agricultural factors for providing such a crop can be specified. More specifically, for example, to the user who has lack of sleep or who ingests too much high-fat food, information relating to a crop for improving disorders of the body which can be caused in such a condition may be provided. Further, the condition of the user may be automatically detected as described above, or similar information may be set through a user operation through the input unit 110.

Further, as described above, the model referring unit 121 may specify the factors for satisfying the conditions based on the premise factors accepted along with the conditions at the condition input unit 111. For example, in the case where the effects of the crop, the factors relating to the crop and the factors relating to the ingester are associated in the model, if the whole of the factors relating to the crop is provided as the premise factors, the model referring unit 121 specifies the behavior factors of the ingester for expressing the effects at the ingester in the case where the ingester ingests the crop satisfying the premise factors (for example, in the case of presenting a lifestyle required for living healthy life assuming that a diet is ideal).

Still further, the operating unit 120 may include a model managing unit 123. As already described above, in the present embodiment, the model defined by the data stored in the database 140 is generated or updated based on the knowledge relating to the effects or the factors, input through the knowledge input unit 113. The model managing unit 123 executes generation or updating of the model based on the knowledge. In the model, for example, as in the above-described example described with reference to FIG. 2, various elements including intermediate factors such as the effects of the crop on the ingester, the components of the crop, the agricultural factors for providing the crop and the behavior factors of the ingester are defined. Further, in the model, translators which convert each element into the class, a mapping engine which associates classes of different elements or associates elements, or the like, are defined. The model managing unit 123 maintains the model in the latest state by constructing a new translator or mapping engine based on the input knowledge or by updating the already constructed translator or mapping engine.

The output unit 130, for example, corresponds to a communication apparatus of the arithmetic apparatus 14, which transmits information to be output to the input/output apparatus 12 or a processor such as a CPU which controls the communication apparatus. For example, the output unit 130 outputs factors relating to the crop or the ingester for satisfying the conditions of the effect specified by the model referring unit 121. For example, output can be executed using text, an image or sound. The output unit 130 provides an output signal corresponding to these output formats or data for output. Further, the output unit 130 may output part or all of the model referred to by the model referring unit 121 as information. For example, because the above-described map or category as described with reference to FIG. 3 to FIG. 6 or the relationship between the components of the crop and the agricultural factors expressed in the map or the category are useful themselves, the map or the category or the relationship may be output through the output unit 130 as information separately from input of the conditions and output of the factor as described above or in association with input/output of these (for example, the model utilized for extraction of the factor is displayed).

(4. Example of utilization of component analysis results)

An example of utilization of the analysis results of the components in the present embodiment will be described next. As already described above, in the present embodiment, the model in which the effects are associated with the factors relating to the crop or the ingester is generated or updated based on the knowledge relating to the effects or the factors. The knowledge includes, for example, the analysis results of the components of the crop. While the above-described association between the component classification of the crop and the agricultural factors described with reference to FIG. 4 to FIG. 6 utilizes results of metabolome analysis, in the present embodiment, it is possible to utilize analysis results of the components of the crop using other various methods. An example of utilization of the analysis results of the components which utilizes light absorbance measurements will be described below as an example of such a method for analyzing the components.

FIG. 8 is a diagram illustrating an example of raw data of absorbance measurements. FIG. 9 is a diagram illustrating an example where the raw data illustrated in FIG. 8 is normalized and the dimension is reduced by expanding the unit width to 100 nm. FIG. 10 is a diagram illustrating an example where the raw data illustrated in FIG. 8 is normalized and the dimension is reduced by expanding the unit width to 50 nm. It should be noted that an absorbance value is regarded as zero in the range where a wavelength is equal to or less than 220 nm in the example illustrated in FIG. 9, and in the range where a wavelength is equal to or less than 250 nm in the example illustrated in FIG. 10.

In the illustrated example, absorbance of ethanolic extract of cabbage cultivated using the conventional farming method (Conventional), the organic farming method (Organic) and the synecological farming method (Synecological) is measured. In the original waveform illustrated in FIG. 8, absorbance measured for each 3 nm is indicated in the range of a light wavelength between 220 nm and 748 nm.

In the waveform illustrated in FIG. 9 and FIG. 10, normalization of dividing an absorbance value of each wavelength by a sum of absorbance values of all wavelengths is performed. The processing of normalization is expressed with the following equation 1 where an absorbance value of a wavelength unit $w_i$ after normalization is $a(w_i)$, and an absorbance value before normalization is $A(w_i)$. It should be noted that the entire wavelength unit is divided into wavelength units (having a wavelength width of 3 nm in the above-described example) of $w_1, w_2, ..., w_n$, and $i, j \in \{1, 2, ..., n\}$.

[Math. 1]

$$a(w_i) = \frac{A(w_i)}{\sum_{j=1}^{n} A(w_j)} \quad \text{Equation 1}$$

For example, when components of crops of the same breed (in the above-described example, cabbages) are compared, there is a case where the moisture evaporates according to the way of storing the sample, and, as a result, components condense. Therefore, it is possible to compare samples while eliminating the influence of condensation of the components by performing normalization as described above.

It should be noted that, while in the analysis of gene expression data, or the like, normalization is performed on specific signals (for example, a gene expression amount), in the above-described example, normalization is performed on the sum of absorbance values. This is because the absorbance waveform is a total of absorbance waveforms of various compounds existing within the sample, and a fixed point cannot be found.

Further, in the waveforms illustrated in FIG. 9 and FIG. 10, the dimension is reduced by expanding the unit width compared to the original waveform (having a wavelength width of 3 nm). The processing of reducing the dimension is expressed with the following equation 2 where an absorbance value of the wavelength $w_i$ is $a(w_i)$ and an absorbance value of the k-th wavelength unit after expansion is $R_k$. It should be noted that the k-th wavelength unit $R_k$ after expansion corresponds to $w_p, w_{p+1}, ..., w_{p+q}$ of the original wavelength unit.

[Math. 2]

$$R_k = \sum_{j=p}^{p+q} a(w_j)$$

Equation 2

For example, in the original waveform illustrated in FIG. 8, in a region less than a wavelength of 400 nm, fine waves (high frequency waves) which are considered as scattering are seen. However, because the observed wavelengths of such fine waves differ every measurement, the fine waves can be considered as noise. Through the processing of reducing the dimension as described above, it is possible to eliminate the influence of noise by averaging the absorbance value in a wider segment and improve an S/N ratio of the measurement results.

Through the normalization and processing of reducing the dimension of the absorbance measurement results as described above, for example, it becomes easy to extract the relationship between the components of the crop and the agricultural factors which is difficult to be recognized in the original waveform. More specifically, the examples illustrated in FIG. 9 and FIG. 10 show that the absorbance value of cabbage cultivated using a synecological farming method (Synecological) is prominently higher than absorbance values of other farming methods in a UV-A region (315 nm to 400 nm) and a UV-B region (280 nm to 315 nm) out of an ultraviolet region of the wavelength.

FIG. 11 is a diagram illustrating an example where another raw data of absorbance measurements is normalized and the dimension is reduced by expanding the unit width to 100 nm. In the illustrated example, absorbance of ethanolic extract of carrots cultivated using the conventional farming method is measured. Further, when samples are acquired (distributed), a sample C-N4 is labeled with "Kintoki carrot", a sample C-N8 is labeled with "Kinbi carrot", and a sample C-N9 is labeled with "Shima carrot". It should be noted that while breeds of carrots are recognized upon measurements for the "Kintoki carrot" and "Shima carrot", the breed of the carrot is not recognized for "Kinbi carrot". Further, while there is no label on a sample C-N7, it is inferred from appearance that the sample C-N7 is Western carrot (typical carrot).

Under assumption as described above, the following can be known from the results of the absorbance measurements illustrated in FIG. 11. First, waveforms of the sample C-N4 and the sample C-N7 stand out in an ultraviolet region of the wavelength between 300 nm and 400 nm (while not illustrated, the waveforms also stand out compared to many other samples). Further, the waveform of the sample C-N4 substantially matches the waveform of the sample C-N7 also in other wavelength regions (while the waveform of the sample C-N7 is illustrated with a dashed line, because the waveform of the sample C-N7 substantially overlaps with the waveform of the sample C-N4, the waveform of the sample C-N7 cannot be seen in the drawing). That is, while there is no label indicating the breed of the sample C-N7, and the sample C-N7 is similar to Western carrot in appearance, the components of the sample C-N7 substantially match the components of Kintoki carrot of the sample C-N4. Therefore, it is estimated that the sample C-N7 is a cross-breed of Kintoki carrot.

On the other hand, waveforms of the sample C-N8 and the sample C-N9 stand out in a region where the wavelength is between 400 nm and 500 nm (while not illustrated, the waveforms also stand out compared to many other samples). Further, the waveform of the sample C-N8 substantially matches the waveform of the sample C-N9 also in other wavelength regions. That is, while the sample C-N8 is different from Shima carrot in appearance, and labeled with "Kinbi carrot" (unidentified), the components of the sample C-N8 substantially match the components of Shima carrot of the sample C-N9. Therefore, it is estimated that "Kinbi carrot" of the sample C-N8 is a breed derived from Shima carrot.

Generally, genome analysis which is expensive and takes a lot of trouble is utilized to identify the breed of the crop. While, as a simpler method, DNA barcoding is utilized in which a variable region of 16S rRNA gene, Rubisco gene relating to a behavior function specific to a plant, or the like, are combined, and a character string of a DNA sequence is used as an ID as is, it is difficult to identify a detailed breed such as a genus, a family and an order with this method.

On the other hand, according to the example of utilization of the absorbance measurement results as described above, it is possible to realize judgment of an evolution of species, process of hybridization and contained pigments by utilizing the results of inexpensive and simple absorbance measurements. More specifically, for example, it is possible to estimate that a crop which appears to be the same breed in appearance (including a label applied upon distribution) includes a cross-breed with another breed or estimate that a crop which appears to be different breed in appearance is derived from a common breed.

The processing on the analysis results of the components as in the above-described example can be executed by, for example, the above-described model managing unit 123. In this case, for example, the model managing unit 123 extracts characteristics of the components by reducing the dimension of a spectrum in analysis results of the components (for example, the results of absorbance measurements as described above) expressed with the spectrum. Further, the model managing unit 123 may extract characteristics of the components by normalizing the sampled spectrum with respect to a sum of sampling values.

(5. Modified example)

A modified example of the present embodiment will be described next.

In the above-described embodiment, a model in which the effects of the crop on the ingester are associated with the factors relating to the crop or the ingester is constructed, and the factors for satisfying the conditions relating to the effect are specified by referring to this model. However, for example, as is clear from the description with reference to FIG. 2, the relationship between the effects and the factors in the model is exchangeable. Therefore, in other embodiments, it is also possible to receive input of the factors relating to the crop or the ingester (such as, for example, agricultural factors for providing the crop and the behavior factors of the ingester) as conditions and specify effects to be realized for such factors.

(6. Hardware configuration)

A hardware configuration of the information processing apparatus according to the embodiment of the present disclosure will be described next with reference to FIG. 12. FIG. 12 is a block diagram illustrating a hardware configuration example of the information processing apparatus according to the embodiment of the present disclosure. The illustrated information processing apparatus 900 can implement, for example, the input/output apparatus, the analyzing apparatus and the storage apparatus in the above-described embodiment.

The information processing apparatus 900 includes a central processing unit (CPU) 901, read only memory (ROM) 903, and random access memory (RAM) 905. In addition, the information processing apparatus 900 may include a host bus 907, a bridge 909, an external bus 911, an interface 913, an input apparatus 915, an output apparatus 917, a storage apparatus 919, a drive 921, a connection port 923, and a communication apparatus 925. Moreover, the information processing apparatus 900 may include an imaging apparatus 933, and a sensor 935, as necessary. The information processing apparatus 900 may include a processing circuit such as a digital signal processor (DSP), an application-specific integrated circuit (ASIC), or a field-programmable gate array (FPGA), alternatively or in addition to the CPU 901.

The CPU 901 serves as an arithmetic processing apparatus and a control apparatus, and controls the overall operation or a part of the operation of the information processing apparatus 900 according to various programs recorded in the ROM 903, the RAM 905, the storage apparatus 919, or a removable recording medium 927. The ROM 903 stores programs, operation parameters, and the like used by the CPU 901. The RAM 905 transiently stores programs used when the CPU 901 is executed, and various parameters that change as appropriate when executing such programs. The CPU 901, the ROM 903, and the RAM 905 are connected with each other via the host bus 907 configured from an internal bus such as a CPU bus or the like. The host bus 907 is connected to the external bus 911 such as a Peripheral Component Interconnect/Interface (PCI) bus via the bridge 909.

The input apparatus 915 is a device operated by a user such as a mouse, a keyboard, a touch panel, a button, a switch, and a lever. The input apparatus 915 may be a remote control device that uses, for example, infrared radiation and another type of radiowave. Alternatively, the input apparatus 915 may be an external connection apparatus 929 such as a mobile phone that supports operations of the information processing apparatus 900. The input apparatus 915 includes an input control circuit that generates input signals on the basis of information which is input by a user to output the generated input signals to the CPU 901. A user inputs various types of data to the information processing apparatus 900 and instructs the information processing apparatus 900 to perform processing operations by operating the input apparatus 915.

The output apparatus 917 includes an apparatus that can report acquired information to a user visually, audibly, or haptically. The output apparatus 917 may be, for example, a display device such as a liquid crystal display (LCD) or an organic electro-luminescence (EL) display, an audio output apparatus such as a speaker or a headphone, or a vibrator. The output apparatus 917 outputs a result obtained through a process performed by the information processing apparatus 900, in the form of video such as text and an image, sounds such as voice and audio sounds, or vibration.

The storage apparatus 919 is an apparatus for data storage that is an example of a storage unit of the information processing apparatus 900. The storage apparatus 919 includes, for example, a magnetic storage device such as a hard disk drive (HDD), a semiconductor storage device, an optical storage device, or a magneto-optical storage device. The storage apparatus 919 stores therein the programs and various data executed by the CPU 901, various data acquired from an outside, and the like.

The drive 921 is a reader/writer for the removable recording medium 927 such as a magnetic disk, an optical disc, a magneto-optical disk, and a semiconductor memory, and built in or externally attached to the information processing apparatus 900. The drive 921 reads out information recorded on the mounted removable recording medium 927, and outputs the information to the RAM 905. The drive 921 writes the record into the mounted removable recording medium 927.

The connection port 923 is a port used to connect devices to the information processing apparatus 900. The connection port 923 may include a Universal Serial Bus (USB) port, an IEEE1394 port, and a Small Computer System Interface (SCSI) port. The connection port 923 may further include an RS-232C port, an optical audio terminal, a High-Definition Multimedia Interface (HDMI) (registered trademark) port, and so on. The connection of the external connection device 929 to the connection port 923 makes it possible to exchange various data between the information processing apparatus 900 and the external connection device 929.

The communication apparatus 925 is a communication interface including, for example, a communication device for connection to a communication network 931. The communication apparatus 925 may be, for example, a communication card for a local area network (LAN), Bluetooth (registered trademark), Wi-Fi, or a wireless USB (WUSB). The communication apparatus 925 may also be, for example, a router for optical communication, a router for asymmetric digital subscriber line (ADSL), or a modem for various types of communication. For example, the communication apparatus 925 transmits and receives signals in the Internet or transits signals to and receives signals from another communication device by using a predetermined protocol such as TCP/IP. The communication network 931 to which the communication apparatus 925 connects is a network established through wired or wireless connection. The communication network 931 may include, for example, the Internet, a home LAN, infrared communication, radio communication, or satellite communication.

The example of the hardware configuration of the information processing apparatus 900 has been described. Each of the structural elements described above may be configured by using a general purpose component or may be configured by hardware specialized for the function of each of the structural elements. The configuration may be changed as necessary in accordance with the state of the art at the time of working of the present disclosure.

(7. Supplement)

The embodiments of the present disclosure may include, for example, the above-described information processing apparatus, the above-described system, the information processing method executed by the information processing apparatus or the system, a program for causing the information processing apparatus to exhibits its function, and a non-transitory physical medium having the program stored therein.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art based on the description of this specification.

Additionally, the present technology may also be configured as below.

(1) An information processing apparatus including:

a condition input unit configured to accept input of conditions relating to an effect of a crop on an ingester; and
a model referring unit configured to specify, by referring to a model in which the effect is associated with a factor relating to the crop or the ingester, the factor for satisfying the conditions.

(2) The information processing apparatus according to (1),
wherein the factor includes an agricultural factor for providing the crop.
(3) The information processing apparatus according to (2),
wherein the agricultural factor includes a breed and a growth environment of the crop.
(4) The information processing apparatus according to (3),
wherein the growth environment includes states of fertilization, vermin/infection control, weed control, tillage, vegetation and biodiversity, a light wavelength, a light amount, a light irradiation period, climate, soil components, a soil state, a dosage amount of fertilizer or pesticide, or a growing period.
(5) The information processing apparatus according to (3) or (4),
wherein the agricultural factor includes a combination of a plurality of breeds or a combination of a plurality of growth environments.
(6) The information processing apparatus according to any one of (2) to (5),
wherein the model includes a component of the crop as an intermediate factor to be associated with the effect and with the agricultural factor.
(7) The information processing apparatus according to (6),
wherein, in the model, the component is classified based on a function.
(8) The information processing apparatus according to (6) or (7),

wherein, in the model, the component is classified based on a metabolic pathway.

(9) The information processing apparatus according to any one of (6) to (8),
wherein, in the model, the component is classified according to whether the component is a natural compound or a synthetic compound.

(10) The information processing apparatus according to (9),
wherein the model referring unit sets a preference relating to the natural compound and the synthetic compound when the model is referred to.

(11) The information processing apparatus according to any one of (6) to (10),
wherein, in the model, the component is classified based on taste or aroma provided to the crop.

(12) The information processing apparatus according to any one of (1) to (11),
wherein the factor includes a behavior factor of the ingester.

(13) The information processing apparatus according to (12),
wherein the condition input unit accepts input of a premise factor relating to the crop along with the conditions, and
the model referring unit specifies the behavior factor for satisfying the conditions based on the premise factor.

(14) The information processing apparatus according to any one of (1) to (13),
wherein the condition input unit accepts input of a premise factor along with the conditions, and
the model referring unit specifies the additional factor for satisfying the conditions based on the premise factor.

(15) The information processing apparatus according to any one of (1) to (14), further including:

a knowledge input unit configured to accept input of knowledge relating to the effect or the factor; and
a model managing unit configured to generate or update the model based on the knowledge.

(16) The information processing apparatus according to (15),
wherein the factor includes an agricultural factor for providing the crop,
the model includes the component of the crop as an intermediate factor to be associated with the effect and with the agricultural factor, and
the knowledge includes an analysis result of the component of the crop for which the agricultural factor is specified.

(17) The information processing apparatus according to (16),
wherein the analysis result of the component is expressed with a spectrum, and
the model managing unit extracts characteristics of the component by reducing a dimension of the spectrum.

(18) The information processing apparatus according to (16) or (17),
wherein the analysis result of the component is expressed with a sampled spectrum, and
the model managing unit extracts characteristics of the component by normalizing the sampled spectrum with respect to a sum of sampling values.

(19) An information processing method including:

accepting input of conditions relating to an effect of a crop on an ingester; and
specifying by a processor, by referring to a model in which the effect is associated with a factor relating to the crop or the ingester, the factor for satisfying the conditions.

(20) A program causing a computer to execute:

a function of accepting input of conditions relating to an effect of a crop on an ingester; and
a function of specifying, by referring to a model in which the effect is associated with a factor relating to the crop or the ingester, the factor for satisfying the conditions.

Reference Signs List

| | |
|---|---|
| 10 | system |
| 12 | input/output apparatus |
| 14 | arithmetic apparatus |
| 16 | storage apparatus |
| 110 | input unit |
| 111 | condition input unit |
| 113 | knowledge input unit |
| 120 | operating unit |

121 model referring unit
123 model managing unit
130 output unit

## Claims

1. An information processing apparatus comprising:

   a condition input unit configured to accept input of conditions relating to an effect of a crop on an ingester; and a model referring unit configured to specify, by referring to a model in which the effect is associated with a factor relating to the crop or the ingester, the factor for satisfying the conditions.

2. The information processing apparatus according to claim 1, wherein the factor includes an agricultural factor for providing the crop.

3. The information processing apparatus according to claim 2, wherein the agricultural factor includes a breed and a growth environment of the crop.

4. The information processing apparatus according to claim 3, wherein the growth environment includes states of fertilization, vermin/infection control, weed control, tillage, vegetation and biodiversity, a light wavelength, a light amount, a light irradiation period, climate, soil components, a soil state, a dosage amount of fertilizer or pesticide, or a growing period.

5. The information processing apparatus according to claim 3, wherein the agricultural factor includes a combination of a plurality of breeds or a combination of a plurality of growth environments.

6. The information processing apparatus according to claim 2, wherein the model includes a component of the crop as an intermediate factor to be associated with the effect and with the agricultural factor.

7. The information processing apparatus according to claim 6, wherein, in the model, the component is classified based on a function.

8. The information processing apparatus according to claim 6, wherein, in the model, the component is classified based on a metabolic pathway.

9. The information processing apparatus according to claim 6, wherein, in the model, the component is classified according to whether the component is a natural compound or a synthetic compound.

10. The information processing apparatus according to claim 9, wherein the model referring unit sets a preference relating to the natural compound and the synthetic compound when the model is referred to.

11. The information processing apparatus according to claim 6, wherein, in the model, the component is classified based on taste or aroma provided to the crop.

12. The information processing apparatus according to claim 1, wherein the factor includes a behavior factor of the ingester.

13. The information processing apparatus according to claim 12, wherein the condition input unit accepts input of a premise factor relating to the crop along with the conditions, and the model referring unit specifies the behavior factor for satisfying the conditions based on the premise factor.

14. The information processing apparatus according to claim 1, wherein the condition input unit accepts input of a premise factor along with the conditions, and

the model referring unit specifies the additional factor for satisfying the conditions based on the premise factor.

**15.** The information processing apparatus according to claim 1, further comprising:

a knowledge input unit configured to accept input of knowledge relating to the effect or the factor; and
a model managing unit configured to generate or update the model based on the knowledge.

**16.** The information processing apparatus according to claim 15,
wherein the factor includes an agricultural factor for providing the crop,
the model includes the component of the crop as an intermediate factor to be associated with the effect and with the agricultural factor, and
the knowledge includes an analysis result of the component of the crop for which the agricultural factor is specified.

**17.** The information processing apparatus according to claim 16,
wherein the analysis result of the component is expressed with a spectrum, and
the model managing unit extracts characteristics of the component by reducing a dimension of the spectrum.

**18.** The information processing apparatus according to claim 16,
wherein the analysis result of the component is expressed with a sampled spectrum, and
the model managing unit extracts characteristics of the component by normalizing the sampled spectrum with respect to a sum of sampling values.

**19.** An information processing method comprising:

accepting input of conditions relating to an effect of a crop on an ingester; and
specifying by a processor, by referring to a model in which the effect is associated with a factor relating to the crop or the ingester, the factor for satisfying the conditions.

**20.** A program causing a computer to execute:

a function of accepting input of conditions relating to an effect of a crop on an ingester; and
a function of specifying, by referring to a model in which the effect is associated with a factor relating to the crop or the ingester, the factor for satisfying the conditions.

FIG. 1

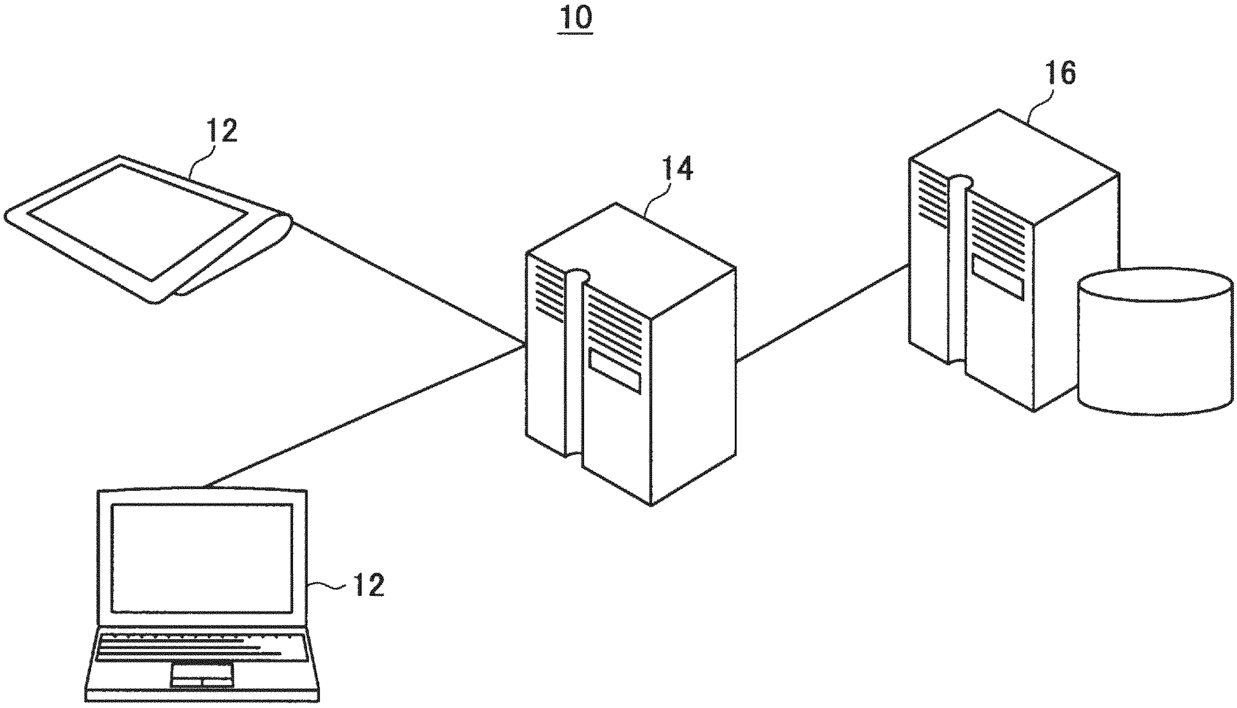

FIG. 2

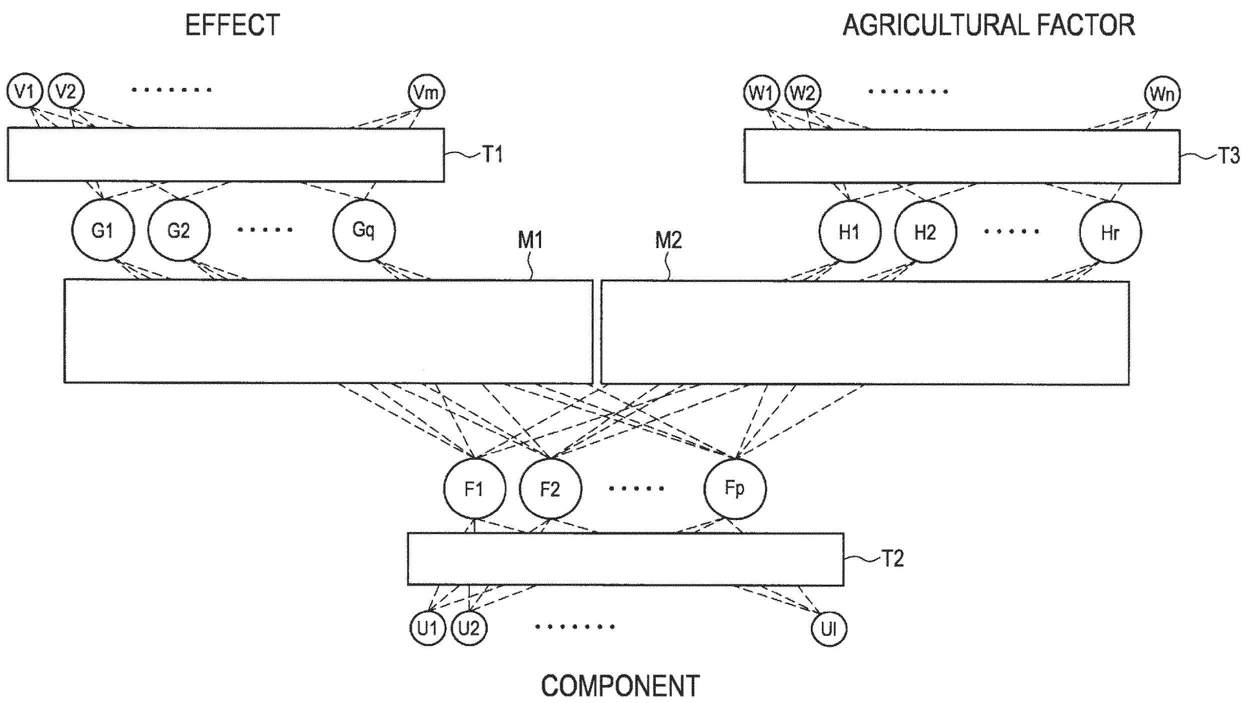
EFFECT
AGRICULTURAL FACTOR
V1
V2
Vm
W1
W2
Wn
T1
T3
G1
G2
Gq
H1
H2
Hr
M1
M2
F1
F2
Fp
T2
U1
U2
Ul
COMPONENT

# FIG. 3

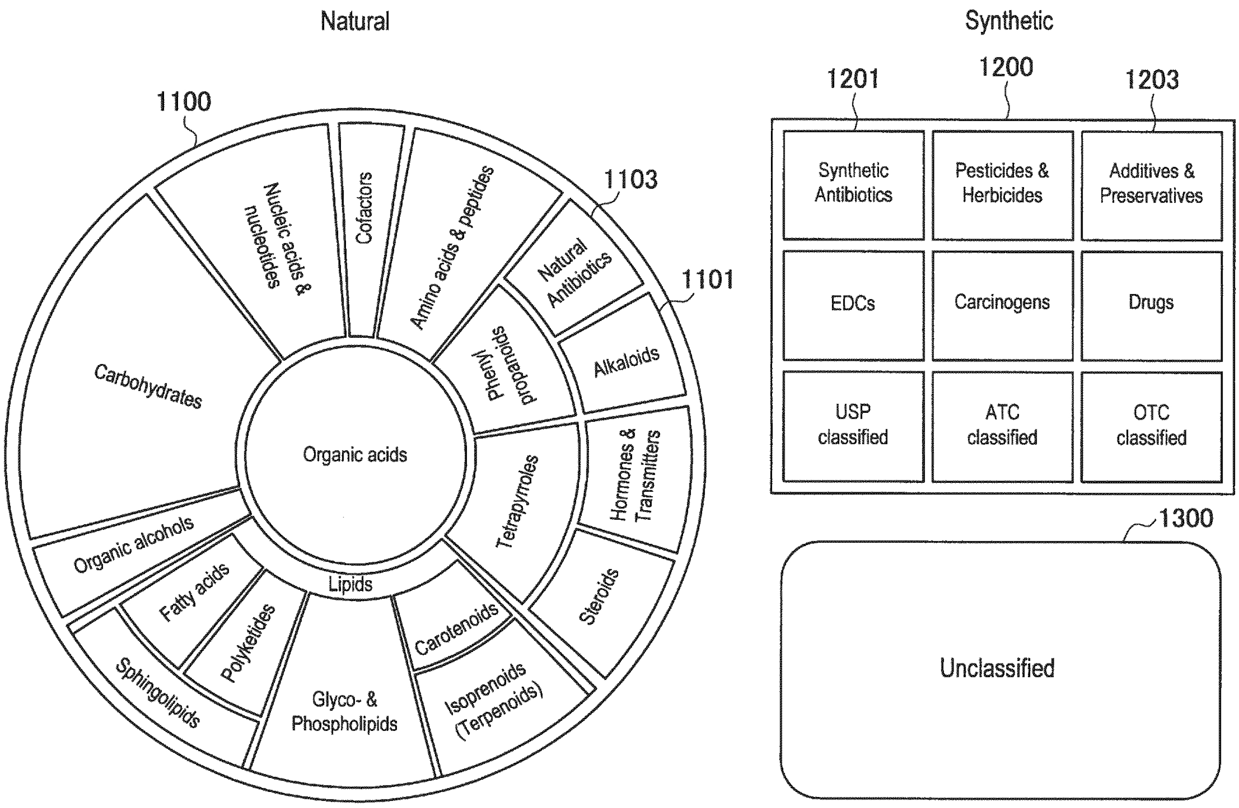
Natural
1100
1103
1101
Nucleic acids & nucleotides
Cofactors
Amino acids & peptides
Natural Antibiotics
Alkaloids
Phenyl propanoids
Carbohydrates
Organic acids
Hormones & Transmitters
Tetrapyrroles
Organic alcohols
Fatty acids
Lipids
Steroids
Polyketides
Carotenoids
Sphingolipids
Glyco- & Phospholipids
Isoprenoids (Terpenoids)
Synthetic
1201
1200
1203
Synthetic Antibiotics
Pesticides & Herbicides
Additives & Preservatives
EDCs
Carcinogens
Drugs
USP classified
ATC classified
OTC classified
1300
Unclassified

FIG. 4

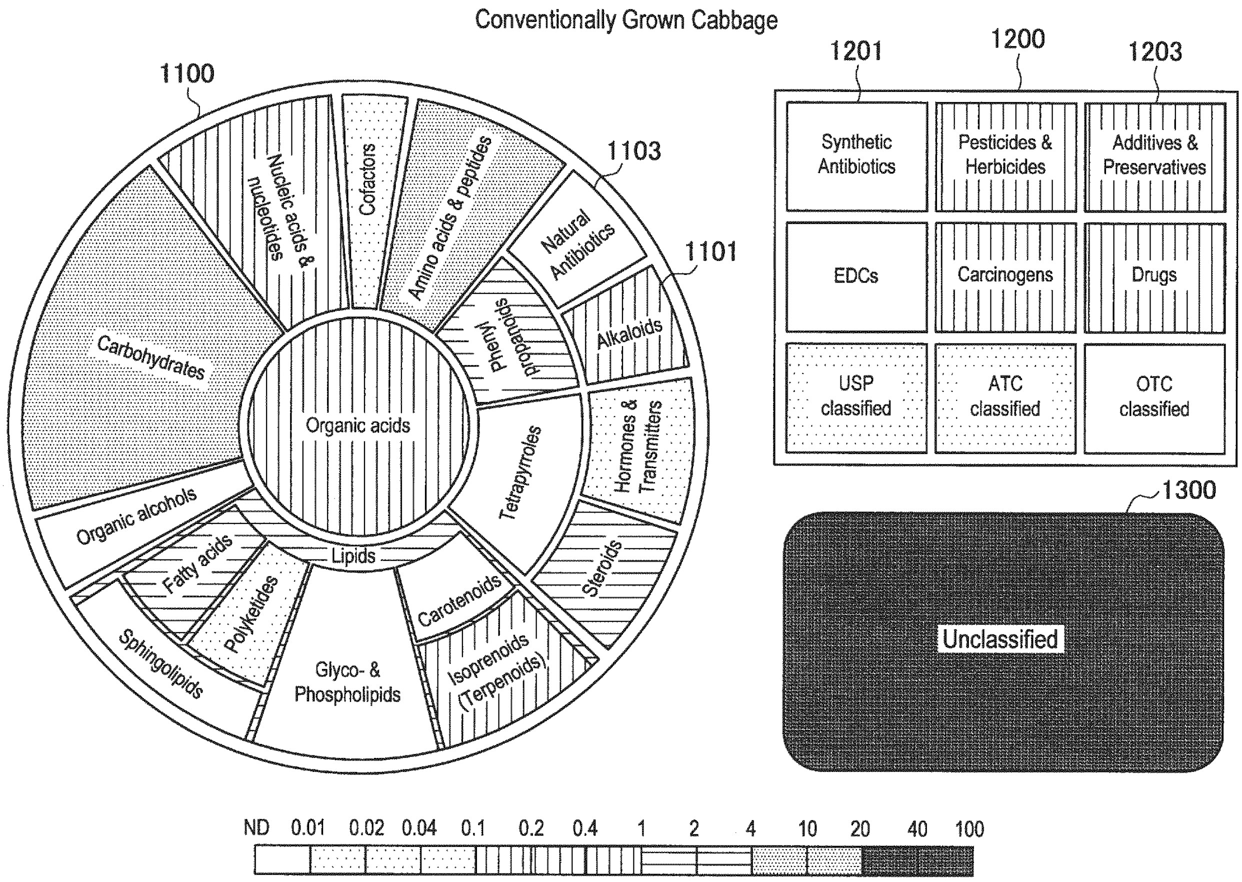
Conventionally Grown Cabbage
1100
1103
1101
1200
1201
1203
1300
Nucleic acids & nucleotides
Cofactors
Amino acids & peptides
Natural Antibiotics
Alkaloids
Phenyl propanoids
Carbohydrates
Organic acids
Tetrapyrroles
Hormones & Transmitters
Steroids
Organic alcohols
Fatty acids
Lipids
Polyketides
Sphingolipids
Glyco- & Phospholipids
Carotenoids
Isoprenoids (Terpenoids)
Synthetic Antibiotics
Pesticides & Herbicides
Additives & Preservatives
EDCs
Carcinogens
Drugs
USP classified
ATC classified
OTC classified
Unclassified
ND 0.01 0.02 0.04 0.1 0.2 0.4 1 2 4 10 20 40 100

FIG. 5

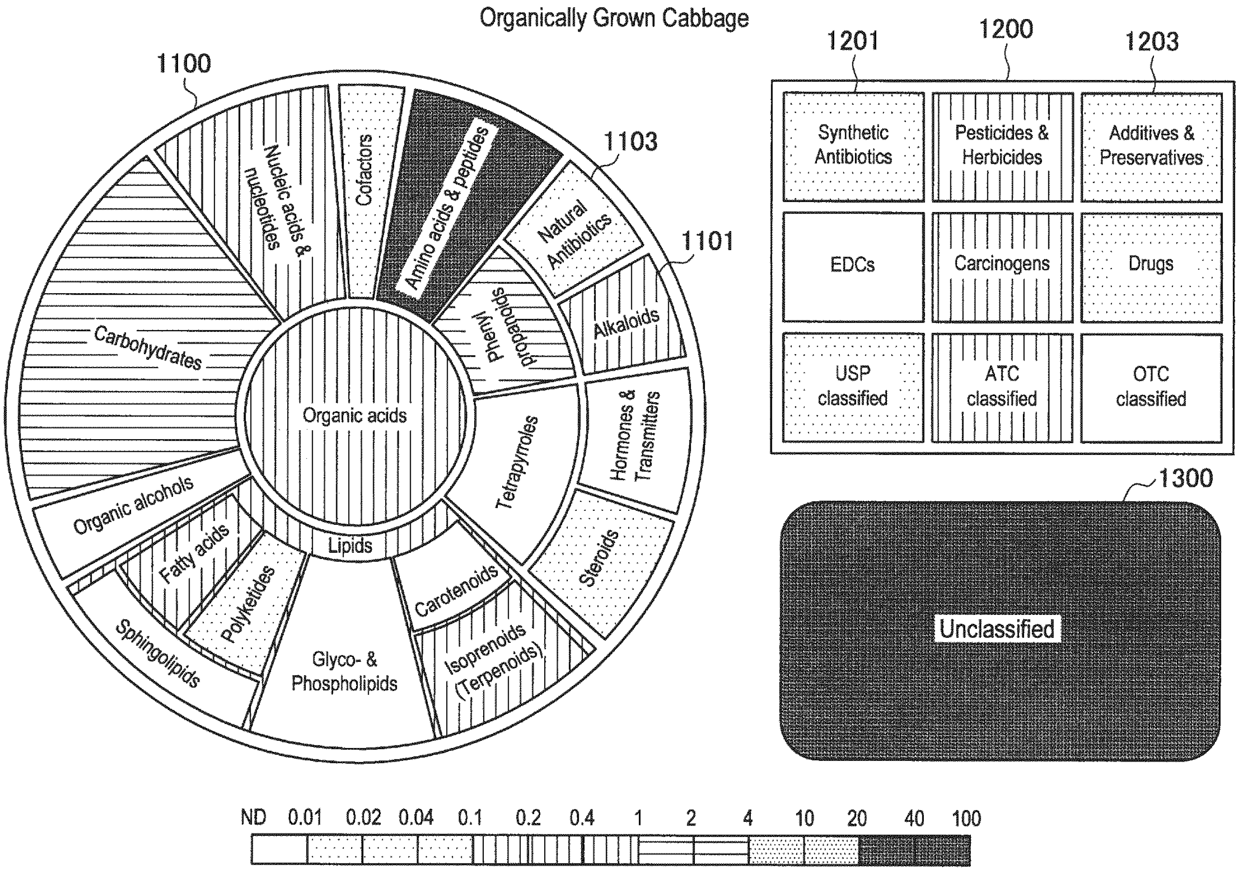
Organically Grown Cabbage
1100
1103
1101
1200
1201
1203
1300
Nucleic acids & nucleotides
Cofactors
Amino acids & peptides
Natural Antibiotics
Alkaloids
Phenyl propanoids
Carbohydrates
Organic acids
Hormones & Transmitters
Tetrapyrroles
Organic alcohols
Fatty acids
Lipids
Polyketides
Steroids
Carotenoids
Sphingolipids
Glyco- & Phospholipids
Isoprenoids (Terpenoids)
Synthetic Antibiotics
Pesticides & Herbicides
Additives & Preservatives
EDCs
Carcinogens
Drugs
USP classified
ATC classified
OTC classified
Unclassified
ND 0.01 0.02 0.04 0.1 0.2 0.4 1 2 4 10 20 40 100

FIG. 6

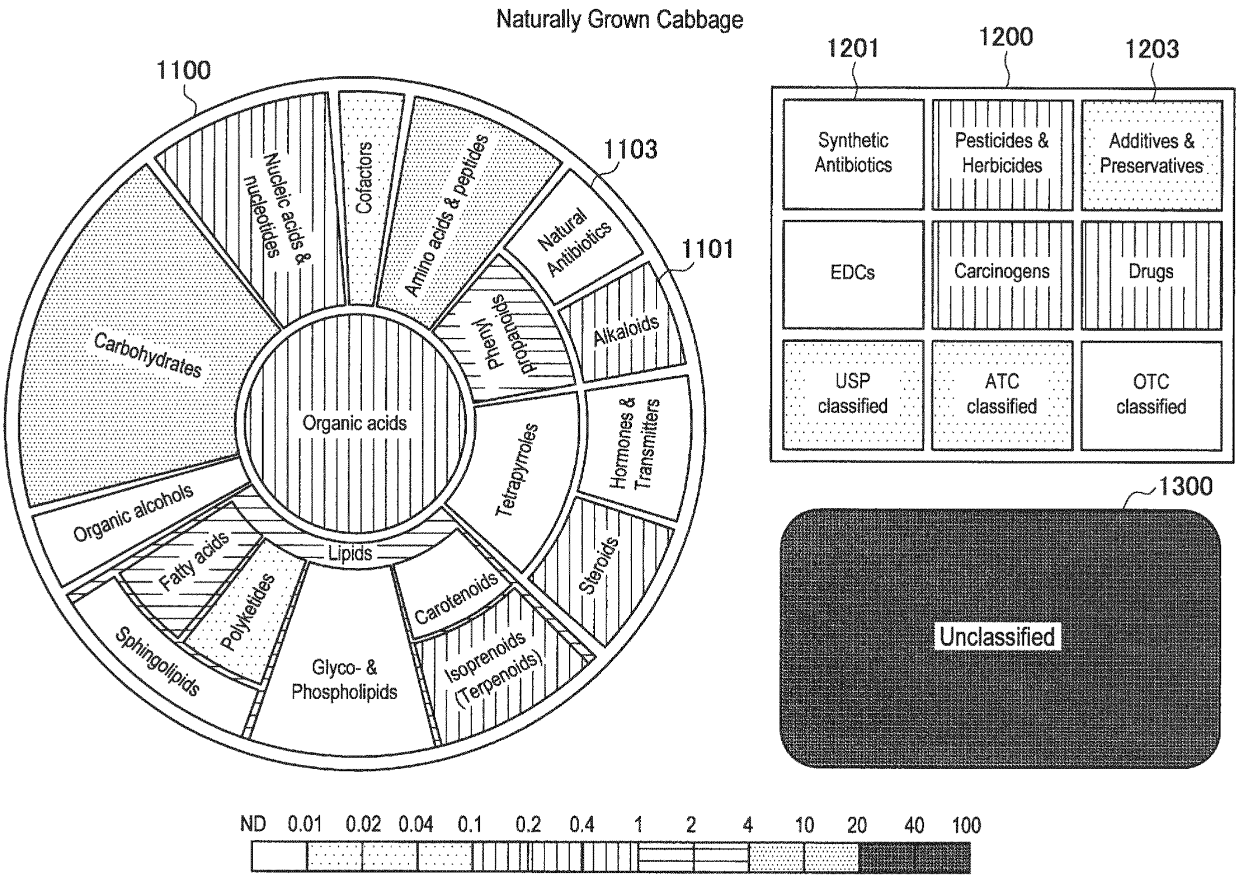
Naturally Grown Cabbage
1100
1103
1101
Nucleic acids & nucleotides
Cofactors
Amino acids & peptides
Natural Antibiotics
Alkaloids
Phenyl propanoids
Carbohydrates
Organic acids
Tetrapyrroles
Hormones & Transmitters
Organic alcohols
Fatty acids
Lipids
Polyketides
Carotenoids
Steroids
Sphingolipids
Glyco- & Phospholipids
Isoprenoids (Terpenoids)
1201
1200
1203
Synthetic Antibiotics
Pesticides & Herbicides
Additives & Preservatives
EDCs
Carcinogens
Drugs
USP classified
ATC classified
OTC classified
1300
Unclassified
ND 0.01 0.02 0.04 0.1 0.2 0.4 1 2 4 10 20 40 100

FIG. 7

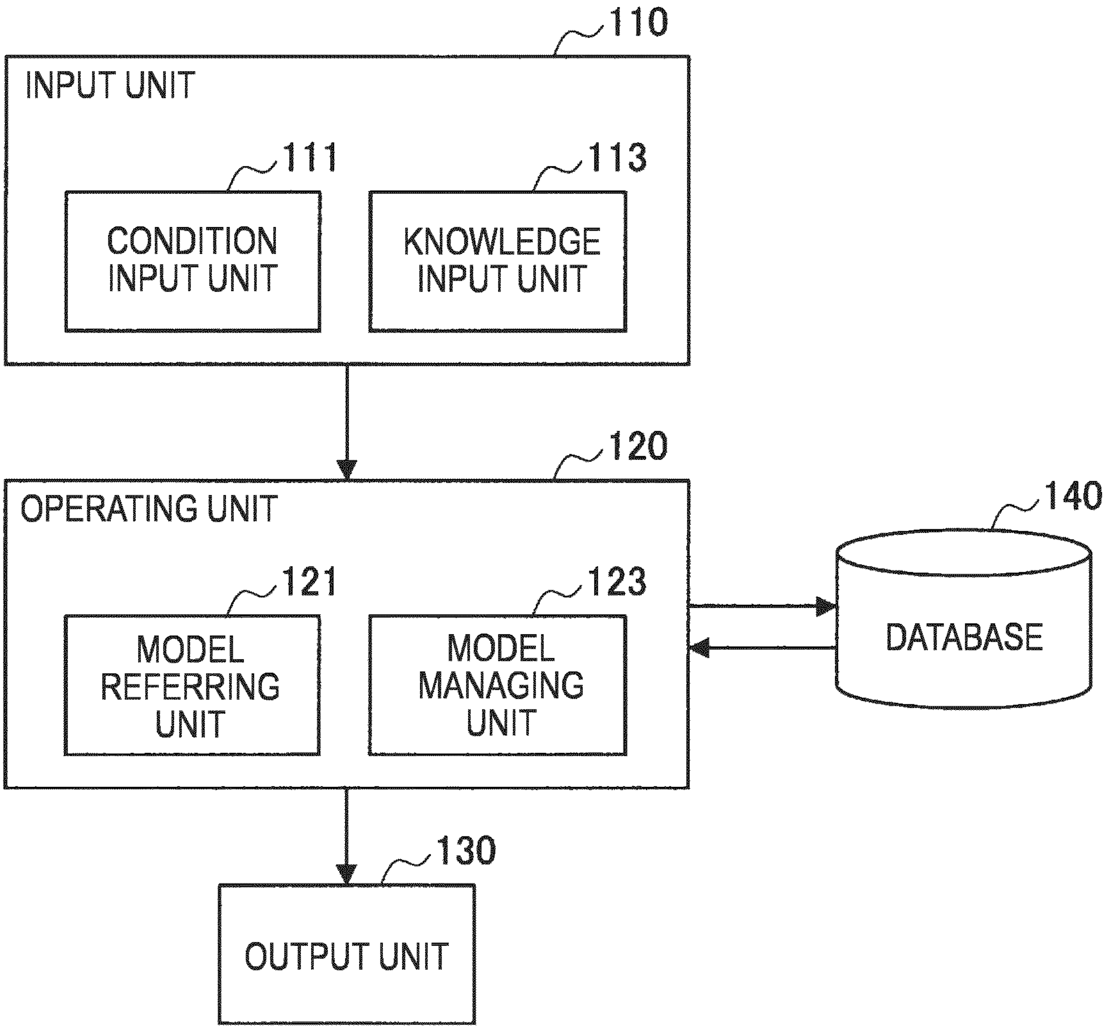
110
INPUT UNIT
111
CONDITION INPUT UNIT
113
KNOWLEDGE INPUT UNIT
120
OPERATING UNIT
121
MODEL REFERRING UNIT
123
MODEL MANAGING UNIT
140
DATABASE
130
OUTPUT UNIT

FIG. 8

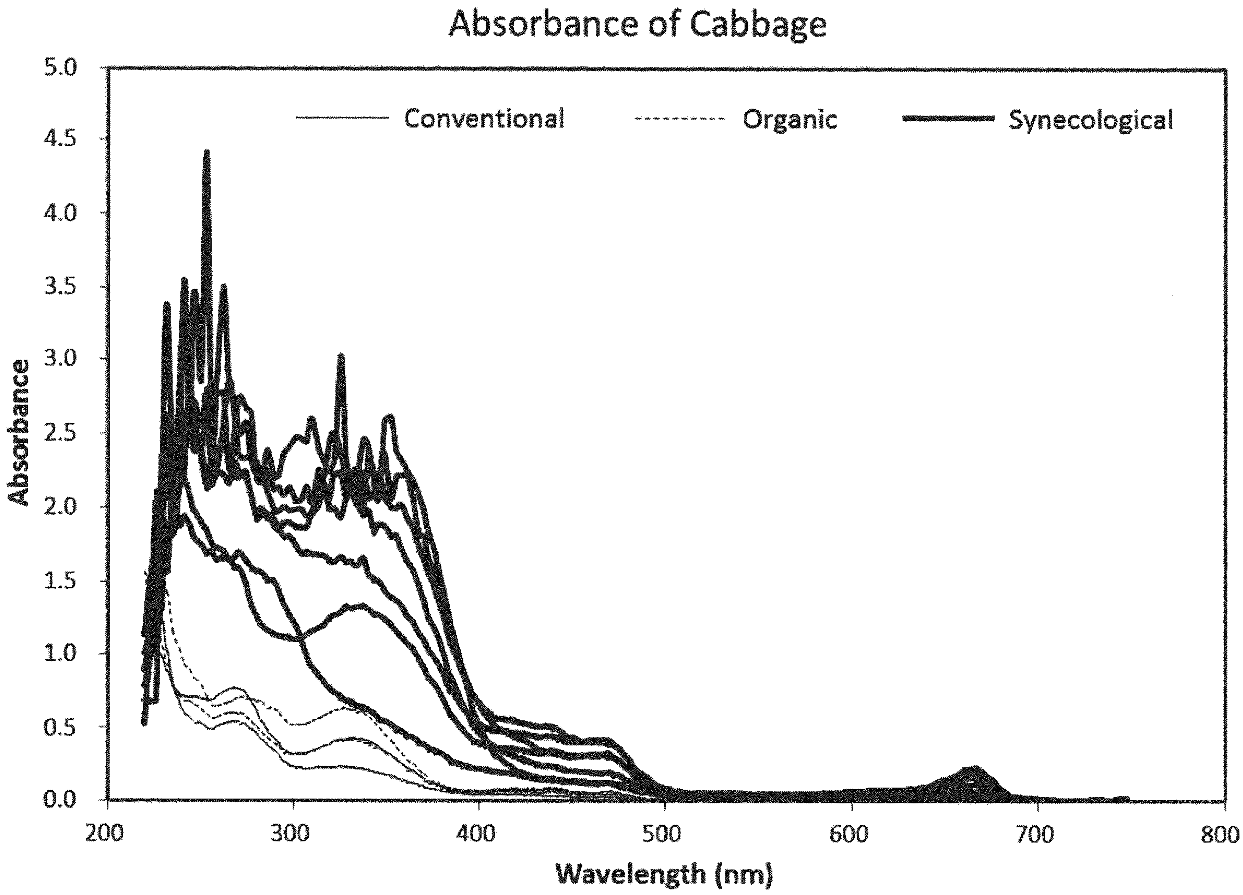
Absorbance of Cabbage
Conventional
Organic
Synecological
Absorbance
5.0
4.5
4.0
3.5
3.0
2.5
2.0
1.5
1.0
0.5
0.0
200
300
400
500
600
700
800
Wavelength (nm)

FIG. 9

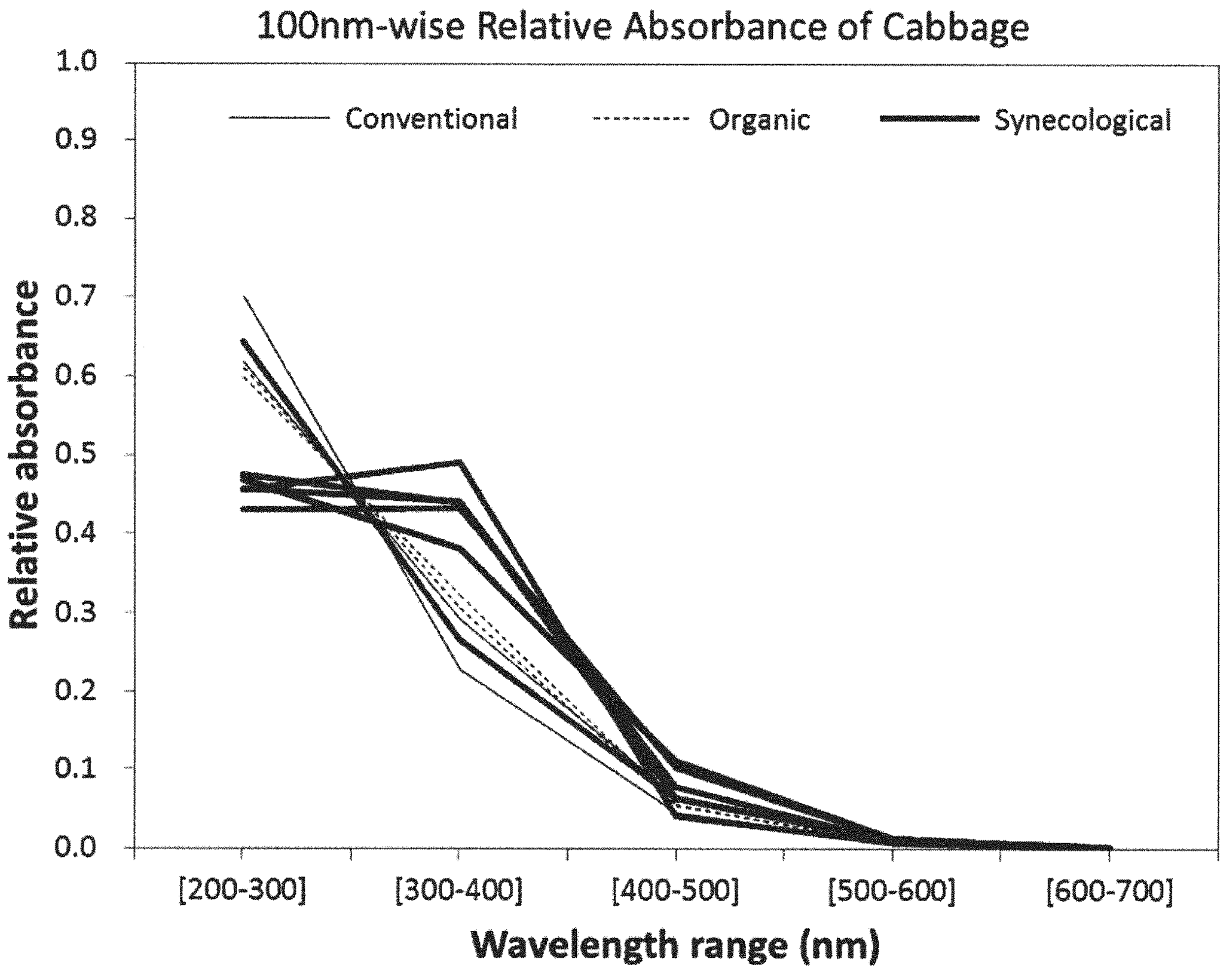
100nm-wise Relative Absorbance of Cabbage
Conventional
Organic
Synecological
Relative absorbance
1.0
0.9
0.8
0.7
0.6
0.5
0.4
0.3
0.2
0.1
0.0
[200-300]
[300-400]
[400-500]
[500-600]
[600-700]
Wavelength range (nm)

FIG. 10

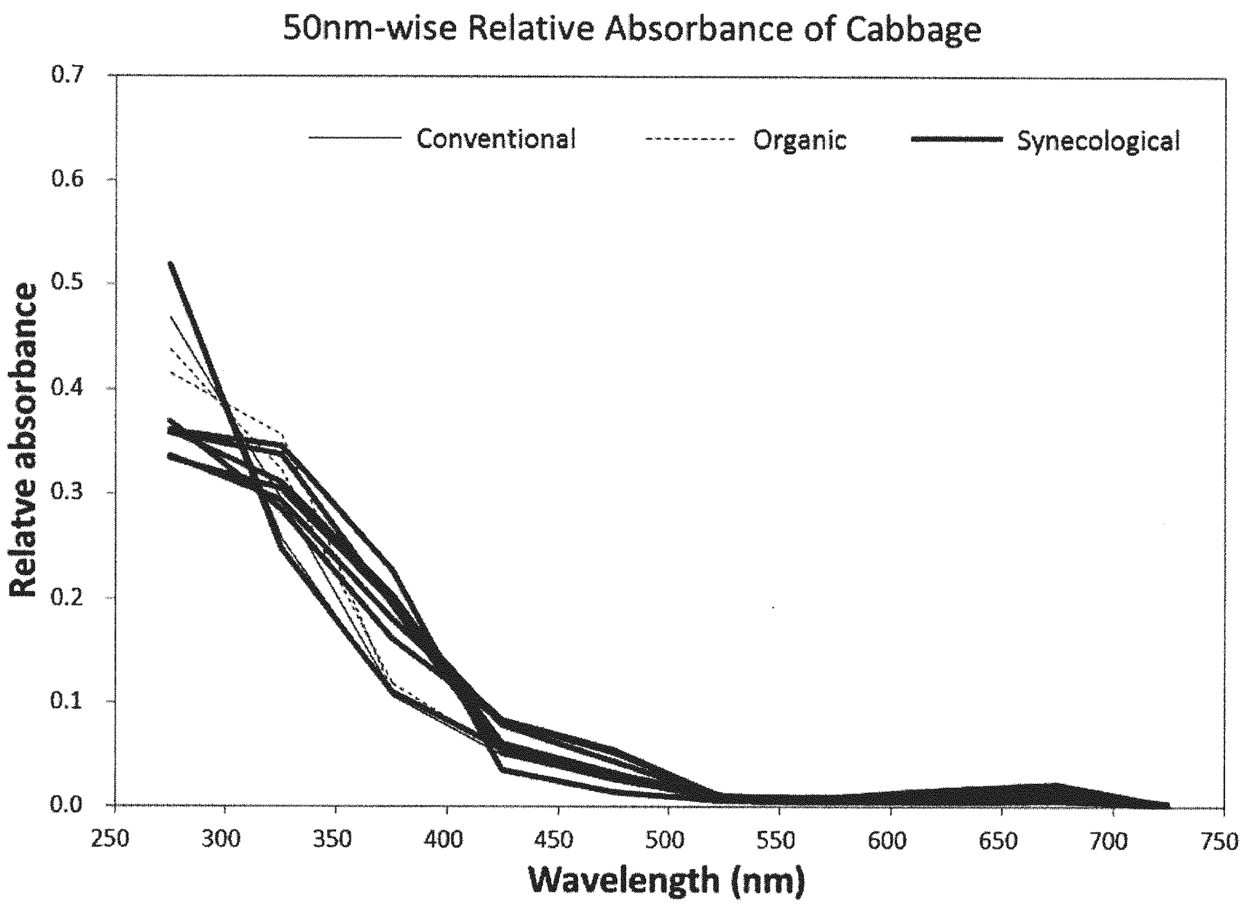
50nm-wise Relative Absorbance of Cabbage
Conventional
Organic
Synecological
Relatve absorbance
0.0
0.1
0.2
0.3
0.4
0.5
0.6
0.7
250
300
350
400
450
500
550
600
650
700
750
Wavelength (nm)

FIG. 11

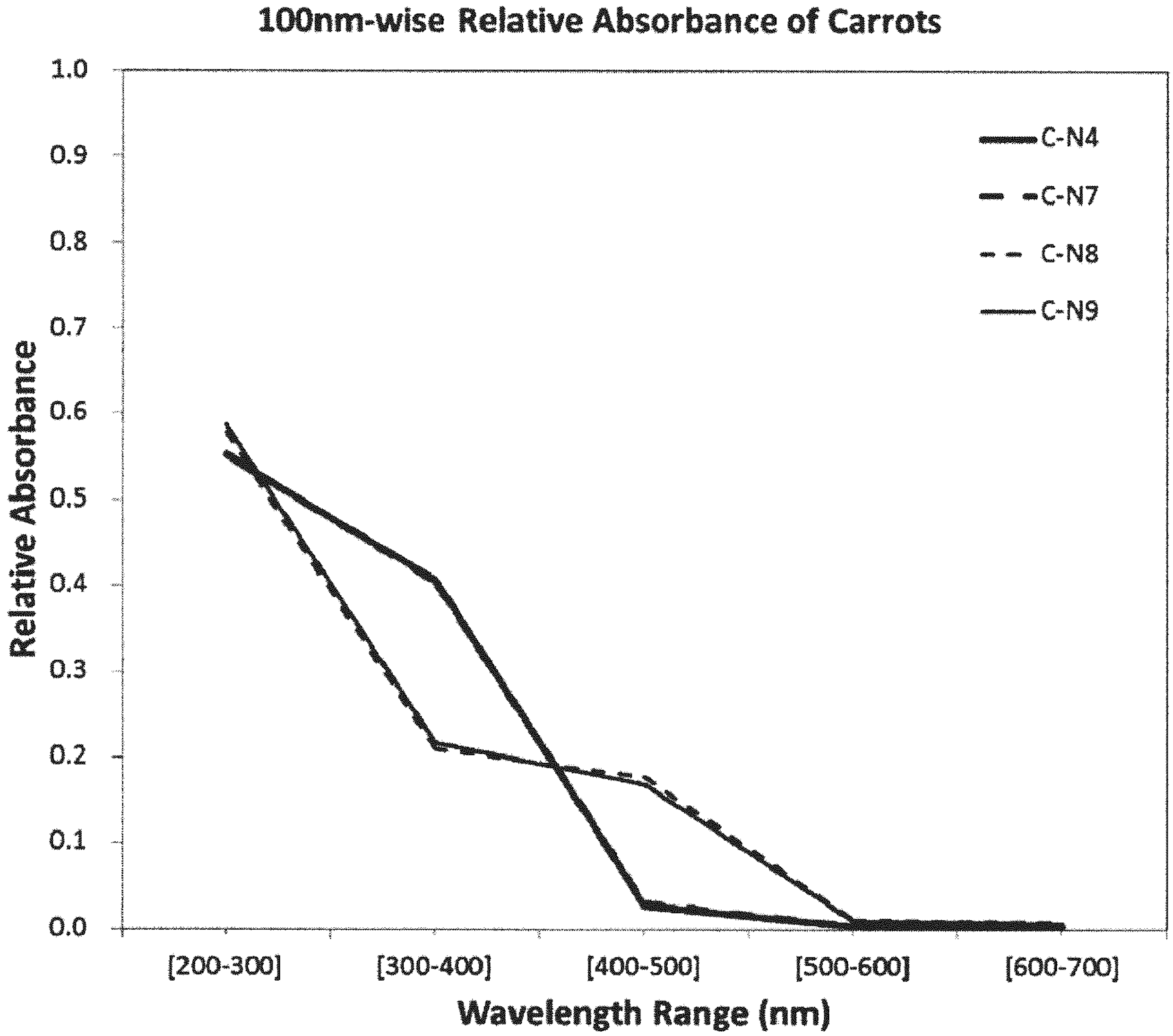
100nm-wise Relative Absorbance of Carrots
Relative Absorbance
1.0
0.9
0.8
0.7
0.6
0.5
0.4
0.3
0.2
0.1
0.0
C-N4
C-N7
C-N8
C-N9
[200-300]
[300-400]
[400-500]
[500-600]
[600-700]
Wavelength Range (nm)

FIG. 12

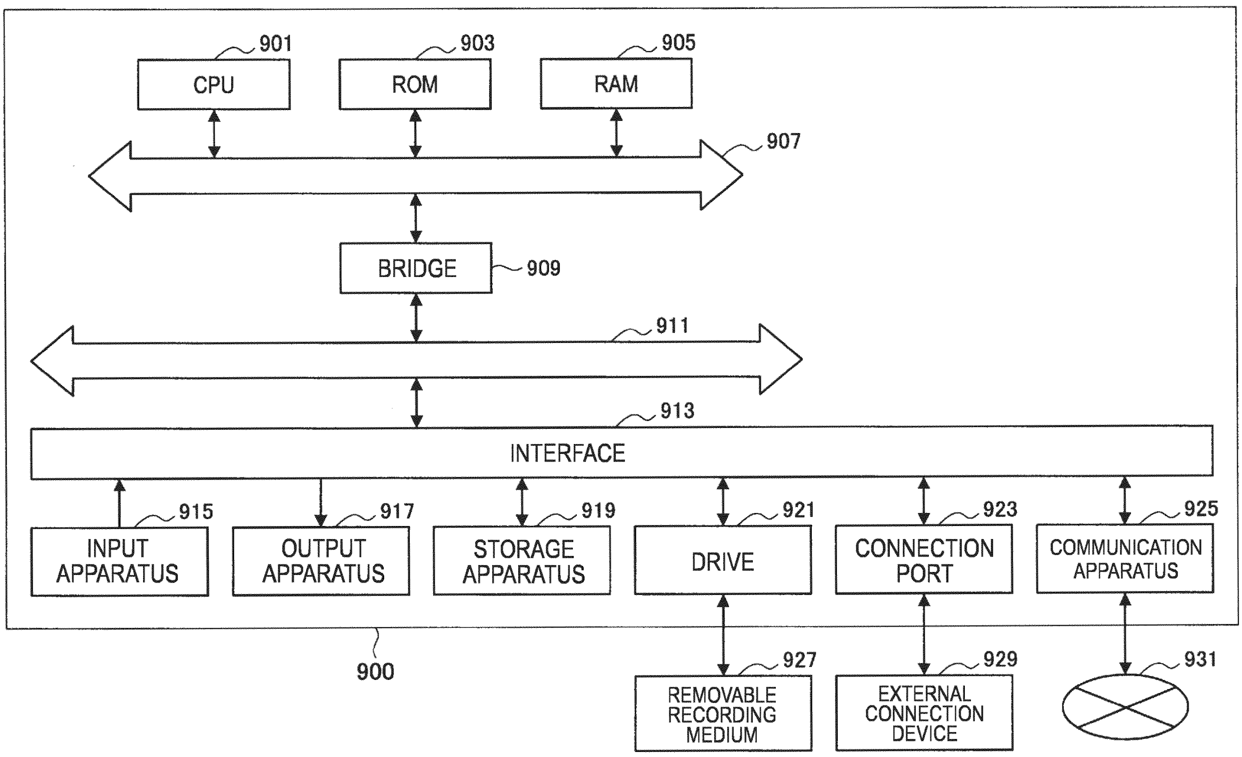
901
CPU
903
ROM
905
RAM
907
BRIDGE
909
911
913
INTERFACE
915
INPUT APPARATUS
917
OUTPUT APPARATUS
919
STORAGE APPARATUS
921
DRIVE
923
CONNECTION PORT
925
COMMUNICATION APPARATUS
900
927
REMOVABLE RECORDING MEDIUM
929
EXTERNAL CONNECTION DEVICE
931

## INTERNATIONAL SEARCH REPORT

International application No.
PCT/JP2015/068838

A. CLASSIFICATION OF SUBJECT MATTER
*A01G7/00*(2006.01)i, *G06Q50/02*(2012.01)i

According to International Patent Classification (IPC) or to both national classification and IPC

B. FIELDS SEARCHED

Minimum documentation searched (classification system followed by classification symbols)
A01G7/00, G06Q50/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2001-88915 A (Kabushiki Kaisha Nippon System Academy), 03 April 2001 (03.04.2001), entire text; all drawings (Family: none) | 1-20 |
| X | JP 2001-265982 A (Jeom Doo KIM), 28 September 2001 (28.09.2001), entire text; all drawings & GB 2365165 A & GB 29560 D0 & DE 10039200 A & KR 10-2001-0006788 A & CN 1313566 A | 1-20 |
| A | JP 2009-230393 A (NEC Corp.), 08 October 2009 (08.10.2009), entire text; all drawings (Family: none) | 1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 September 2015 (17.09.15) | 06 October 2015 (06.10.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/068838

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-302105 A (Norinsuisansho Tohoku Nogyo Shikenjo-cho), 13 November 1998 (13.11.1998), entire text; all drawings (Family: none) | 1-20 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009020037 A